# EUROPÄISCHE PATENTSCHRIFT

(11) **EP 0 447 778 B1**
(45) Veröffentlichungstag und Bekanntmachung des Hinweises auf die Patenterteilung: **18.10.1995**
(21) Anmeldenummer: 91101649.1
(22) Anmeldetag: 07.02.1991
(51) Int. Cl.: C07C 43/178, C07C 309/09, C11D 1/14, A61K 7/00

(54) **Vinylpolyetheralkohole**
Vinyl polyether alcohols
Polyétheralcools vinyliques

(30) Priorität: 16.02.1990 DE 4004883
(43) Veröffentlichungstag der Anmeldung: 25.09.1991
(73) Patentinhaber: BASF Aktiengesellschaft, 67063 Ludwigshafen (DE)
(72) Erfinder: Fischer, Martin, Dr., W-6700 Ludwigshafen (DE); Baur, Richard, Dr., W-6704 Mutterstadt (DE); Diessel, Paul, W-6704 Mutterstadt (DE)

(56) Entgegenhaltungen:
- EP-A- 0 149 173
- EP-A- 0 155 710
- EP-A- 0 174 610
- EP-A- 0 244 841
- EP-A- 0 299 360
- FR-A- 2 263 235
- GB-A- 715 913

## Beschreibung

Die vorliegende Erfindung betrifft neue Vinylpolyetheralkohole der allgemeinen Formel I 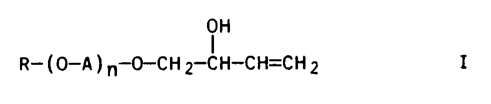
in der
- R: für C₁-C₂₅-Alkyl-, C₂-C₂₅-Alkenyl- oder Alkylarylgruppen mit insgesamt bis zu 20 C-Atomen steht,
- A: eine 1,2-Alkylengruppe mit 2 bis 4 C-Atomen bezeichnet und
- n: eine Zahl von 1 bis 20 bedeutet,
sowie ein Verfahren zur Herstellung der Verbindungen I, ihre Verwendung als oberflächenaktive Verbindungen in oberflächenaktiven Zubereitungen und diese oberflächenaktiven Zubereitungen selbst.

Weiterhin betrifft die vorliegende Erfindung ein Verfahren zur Herstellung von Polyethersulfonaten der allgemeinen Formel III 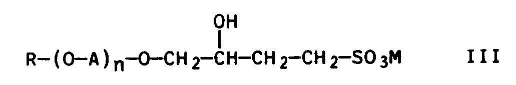
in der M für Wasserstoff, ein Alkalimetall oder Ammonium steht, aus Vinylpolyetheralkoholen I sowie die Verwendung der Verbindungen III als oberflächenaktive Verbindungen in Wasch-, Reinigungs- und Körperpflegemitteln und diese Mittel selbst.

Polyglykolethersulfonate, wie sie beispielsweise in der DE-A 37 35 056 (1) beschrieben sind, stellen wichtige oberflächenaktive Verbindungen dar, die als Tenside auf verschiedensten technischen Gebieten Anwendung finden. übliche Herstellungsverfahren für derartige Verbindungen sind beispielsweise die Chlorierung der entsprechenden Polyglykoletheralkohole mit Mitteln wie Thionylchlorid oder Phosgen und die anschließende Umsetzung mit Alkalimetallsulfiten; dies ist ein aufwendiger Weg, der außerdem Korrosions-, Entsorgungs- und Toxizitätsprobleme mit sich bringt. Daher sind neue und vereinfachte Synthesewege, die zu solchen Polyethersulfonaten führen, von großem Interesse, insbesondere für solche Polyethersulfonate, die noch zusätzliche funktionelle Gruppen wie Hydroxyl im Molekül in Nachbarschaft zur Sulfonatgruppe enthalten.

Vinyloxiran als Synthesebaustein ist z.B. aus der JP-AS 87/45 851 (2) für die Herstellung von Hydroxy-hydrocarbylethern aus Alkoholen oder Phenolen und Epoxiden bekannt.

Die Polyethersulfonate III werden in den Schriften DE-A 28 54 826 (3), US-A 4 421 168 (4) und US-A 4 463 806 (5) als Hilfsmittel bei der Erdölgewinnung aus unterirdischen Lagerstätten empfohlen.

Polyethersulfate, wie sie in der Technischen Information TI/P 2759d über Lutensit®-AS-Marken der BASF Aktiengesellschaft vom Juni 1983 (6) beschrieben sind, werden häufig als Tenside in Wasch-, Reinigungs- und Körperpflegemitteln eingesetzt.

Aufgabe der vorliegenden Erfindung war es, einen einfachen und effizienten Syntheseweg für die Polyethersulfonate III aufzufinden.

Demgemäß wurden die eingangs definierten Vinylpolyetheralkohole I als Zwischenprodukte für die Herstellung der Polyethersulfonate III gefunden, wobei die Verbindungen I selber oberflächenaktive Verbindungen mit wertvollen anwendungstechnischen Eigenschaften darstellen.

Die Vinylpolyetheralkohole I weisen als hydrophobe Reste R geradkettige oder verzweigte C₁-C₂₅-Alkylgruppen, geradkettige oder verzweigte C₂-C₂₅-Alkenylgruppen oder Alkylarylgruppen mit insgesamt bis zu 20 C-Atomen auf.

Beispiele für C₁-C₂₅-Alkylgruppen sind Methyl, Ethyl, Propyl, Butyl, Hexyl, Octyl, Isooctyl, 2-Ethylhexyl, Nonyl, Isononyl, Decyl, Undecyl, Dodecyl, Tridecyl, Isotridecyl, Myristyl, Cetyl, Stearyl und Eicosyl.

Beispiele für C₂-C₂₅-Alkenylgruppen sind Vinyl, 1-Propenyl, 2-Propenyl, Oleyl, Linolyl und Linolenyl.

Beispiele für Alkylarylgruppen mit insgesamt bis zu 20 C-Atomen sind Tolyl, Methylnaphthyl, Xylyl, Mesityl, Cumyl, Ethylphenyl, Propylphenyl, Butylphenyl, Hexylphenyl, Octylphenyl, 2-Ethylhexylphenyl, Nonylphenyl, Decylphenyl, Dodecylphenyl und Myristylphenyl. Die Substituenten an den aromatischen Ringsystemen können in beliebiger Position stehen.

Bevorzugte Verbindungen I sind solche, bei denen R für längerkettige Alkylgruppen steht, insbesondere für C₈-C₂₀-Alkylgruppen. Solchen Resten R liegen beispielsweise natürlich vorkommende Fettalkohole oder synthetisch hergestellte Alkohole zugrunde; letztere werden üblicherweise durch die Oxosynthese oder die Ziegler-Synthese hergestellt und sind dann in der Regel Gemische verschiedener Isomerer und benachbarter Homologer, beispielsweise C₉-/C₁₁- oder C₁₃-/C₁₅-Oxoalkohole und C₈-/C₁₀-, C₁₂-/C₁₄- oder C₁₀-/C₂₀-Ziegler-Alkohole.

Die 1,2-Alkylengruppe A bezeichnet insbesondere die Ethylen-, daneben aber auch die Propylen-, 1,2-Butylen- und 2,3-Butylengruppe.

Der Alkoxylierungsgrad n liegt zwischen 1 und 20, vorzugsweise 2 und 15, insbesodere 3 und 10. Der Wert für n stellt üblicherweise einen Durchschnittswert dar.

Die Vinylpolyetheralkohole I werden zweckmäßigerweise durch Umsetzung von Polyetheralkoholen der allgemeinen Formel II

R-(O-A)ₙ-OH II

mit Vinyloxiran in Gegenwart einer Base hergestellt.

Als Base verwendet man in der Regel ein Alkali- oder Erdalkalimetallhydroxid wie Natrium-, Kalium-, Calcium- oder Bariumhydroxid oder das Alkoholat eines niedrigsiedenden Alkohols wie Natriummethylat, Natriumethylat oder Kalium-tert.-butylat. Man verwendet hiervon katalytische Mengen, etwa 0,1 bis 5 mol-%, bezogen auf II. Bei höhersiedenden Alkoholen II ist es vorteilhaft, mit der Base eingebrachte niedrigsiedende Lösungsmittel wie Wasser oder Alkohol vor der Umsetzung mit Vinyloxiran abzudestillieren.

Die Umsetzung mit Vinyloxiran erfolgt üblicherweise bei Temperaturen von 50°C bis 180°C, vorzugweise 100 bis 160°C, bei Normaldruck oder bei erhöhtem Druck bis etwa 10 bar. Man kann den Alkohol II zusammen mit Vinyloxiran und der Base gemeinsam auf die notwendige Reaktionstemperatur erhitzen oder das Vinyloxiran dem erhitzten Reaktionsgemisch zudosieren, wobei sich bei der zweiten Möglichkeit die Kontrolle der Abfuhr der Reaktionswärme unproblematischer gestaltet.

Pro Mol Alkohol II setzt man in der Regel 1 bis 2 mol, vorzugsweise 1 bis 1,5 mol Vinyloxiran zu. Eventuell verbleibende Reste an nicht umgesetztem Vinlyoxiran können abdestilliert oder durch Strippen mit einem Inertgas wie Stickstoff entfernt werden.

Die Umsetzung von II mit Vinyloxiran kann auch in Gegenwart eines gegen Vinyloxiran und gegen Alkali inerten Lösungsmittels wie z.B. Tetrahydrofuran, Methyl-tert.-butylether, Dioxan, Toluol oder Xylol durchgeführt werden.

Man kann die Umsetzung von 11 mit Vinyloxiran diskontinuierlich oder kontinuierlich in einer Rührkesselkaskade oder einem Rohrreaktor durchführen.

Weiterhin betrifft die Erfindung ein Verfahren zur Herstellung von Polyethersulfonaten III aus Vinylpolyetheralkoholen I durch Umsetzung der Verbindungen I mit einem Alkalimetall- oder Ammoniumsulfit, -hydrogensulfit oder -disulfit oder Mischungen hiervon. Als Alkalimetalle treten vornehmlich Natrium und Kalium auf.

Besonders gut eignen sich für diese Umsetzung Alkalimetallhydrogensulfite wie Natrium- oder Kaliumhydrogensulfit, z.B. auch in Form von technischen Lösungen, oder Mischungen von Alkalimetallhydrogensulfiten mit Alkalimetallsulfiten. Arbeitet man nämlich in Gegenwart von Luftsauerstoff, wird ein Teil des Hydrogensulfits zu Hydrogensulfat oxidiert, welches mit Sulfit Hydrogensulfit nachbildet, so daß das unter den üblichen Reaktionsbedingungen reaktionsträgere Sulfit ebenfalls für die Addition an I genutzt werden kann. Das molare Verhältnis von Sulfit zu Hydrogensulfit beträgt hierbei vorteilhafterweise etwa 1 : 2,5 bis etwa 1 : 1.

Die Sulfitaddition erfolgt in der Regel bei Temperaturen von 20 bis 130°C, vorzugsweise 50 bis 100°C, bei Normaldruck oder leicht erhöhtem Druck bis etwa 2 bar. Zur Beschleunigung kann man einen Radikalstarter, beispielsweise ein organisches Peroxid wie Dibenzoylperoxid, eine Azoverbindung wie Azobisisobutyronitril oder eine wasserlösliche Peroxoverbindung wie Kaliumperoxodisulfat, zusetzen oder Luft durch das Reaktionsgemisch leiten.

Eine hohe Reaktionsgeschwindigkeit wird u.a. dadurch erzielt, daß man die Vinylverbindung I und das Sulfit, Hydrogensulfit oder Disulfit in dem Reaktionsmedium zumindest teilweise löst. Als Lösungsvermittler hierfür eignen sich vor allem Wasser und wassermischbare Alkohole wie Methanol, Ethanol, n-Propanol, Isopropanol, n-Butanol, sek.-Butanol oder tert.-Butanol.

Pro Mol I setzt man in der Regel 1 bis 3 mol, vorzugsweise 1 bis 1,5 mol Sulfit, Hydrogensulfit, Disulfit oder einer Mischung hiervon ein.

Die Sulfitaddition erfordert unter den verwendeten Bedingungen etwa 1 bis 10 Stunden, in Ausnahmefällen bis zu 50 Stunden. Im Anschluß an die Umsetzung, deren Endpunkt über eine Redoxtitration ermittelt werden kann, wird gegebenenfalls ausgefallenes Salz abfiltriert und Wasser und eventuell vorhandene Lösungsmittel werden abdestilliert. Man erhält so die Polyethersulfonate III in zähflüssiger bis pastöser Form. Will man die freien Sulfonsäuren herstellen, müssen die erhaltenen Salze noch mit Säure umgesetzt werden.

In einer bevorzugten Ausführungsform werden beide Umsetzungsschritte, nämlich die Umsetzung der Polyetheralkohole II mit Vinyloxiran zu Vinylpolyetheralkoholen I und die Sulfitaddition an die Verbindungen I, zu einem Gesamtverfahren für die Herstellung der Polyethersulfonate III kombiniert, was wirtschaftlich besonders vorteilhaft ist, weil das Zwischenprodukt I nicht gereinigt zu werden braucht und im gleichen Reaktionsgefäß weiter umgesetzt werden kann.

Die erfindungsgemäßen Vinylpolyetheralkohole I eignen sich als oberflächenaktive Verbindungen und weisen somit eine Vielzahl von technischen Anwendungsmöglichkeiten auf. Mögliche Einsatzgebiete sind beispielsweise Wasch- und Reinigungsmittel für Haushalt und Industrie, die Galvanotechnik, die photographische Industrie, die Textilindustrie, die Papierindustrie, die Erdölförderung, die pharmazeutische Industrie, die kosmetische Industrie, die Nahrungsmittelindustrie und die Pflanzenernährung.

Weiterhin sind Gegenstand der vorliegenden Erfindung oberflächenaktive Zubereitungen, welche 1 bis 50 Gew.-%, vorzugsweise 1 bis 30 Gew.-% eines Vinylpolyetheralkohols I oder eine Mischung solcher Vinylpolyetheralkohole als oberflächenaktive Verbindung neben üblichen Hilfsstoffen und gegebenenfalls anderen üblichen Tensiden enthalten.

Die Polyethersulfonate III finden Verwendung als oberflächenaktive Verbindungen in Wasch-, Reinigungs- und Körperpflegemitteln.

Gegenstand der vorliegenden Erfindung sind ebenfalls Wasch-, Reinigungs- und Körperpflegemittel, welche 1 bis 50 Gew.-%, vorzugsweise 5 bis 45 Gew.-% eines Polyethersulfonates III oder eine Mischung solcher Polyethersulfonate enthalten.

Die die Verbindungen III als Emulgatoren enthaltenden Körperpflegemittel wie Hautcremes, Lotionen, Gele, Hautöle oder Haarshampoos können als weitere Komponenten kosmetische Öle, weitere übliche Emulgatoren, Lichtschutzmittel, Konservierungsmittel, Parfüms und sonstige übliche Hilfsstoffe enthalten.

Die vorliegende Erfindung zeigt einen einfachen und wirtschaftlich attraktiven Herstellungsweg für die Polyethersulfonate III ausgehend von technisch leicht zugänglichen Produkten auf, der weitgehend von den für die üblichen Synthesewege charakteristischen Toxikologie-, Umwelt- und Entsorgungsproblemen frei ist.

Die Polyethersulfonate III weisen gerade für Wasch-, Reinigungs- und Körperpflegemittel günstige Tensideigenschaften auf, insbesondere eine gute Hartwasserbeständigkeit und eine hohe Verseifungsstabilität in alkalischem und schwach saurem Medium.

### Herstellungsbeispiele

### Beispiel 1

174 g (0,4 mol) eines mit 5 mol Ethylenoxid umgesetzten C₁₃-/C₁₅-Oxo-alkohols wurden mit 2,4 g einer 30 gew.-%igen methanolischen Natriummethylatlösung (entsprechend 13 mmol NaOCH₃) versetzt und Methanol wurde bei 60°C und 30 mbar abdestilliert. Bei 138 bis 140°C wurden anschließend innerhalb von 3,5 Stunden 31 g (0,44 mol) Vinyloxiran zudosiert. Es wurden 206 g Vinylpolyetheralkohol, entsprechend 100 % Ausbeute, erhalten. Die Hydrierjodzahl betrug 50 und der Trübungspunkt gemäß DIN 53 917 lag bei 52°C.

Der erhaltene Vinylpolyetheralkohol wurde in einer Mischung aus 335 ml Ethanol und 130 ml Wasser bei Raumtemperatur gelöst. Unter Durchleiten von Luft wurde eine Lösung von 30,3 g (0,29 mol) Natriumhydrogensulfit und 17,8 g (0,14 mol) Natriumsulfit in 80 ml Wasser innerhalb von 2 Stunden zugetropft. Anschließend wurden 1 Stunde bei Raumtemperatur und 2 Stunden unter Rückfluß gerührt. Nach Abdestillieren von Wasser und Ethanol bei 60°C und 20 mbar verblieben 254 g Polyethersulfonat, entsprechend 100 % Ausbeute, als zähflüssiges Öl. 1 g des Sulfonates löste sich klar in 100 ml Wasser.

### Beispiel 2

Analog zu Beispiel 1 wurden 138 g (0,4 mol) eines mit 3 mol Ethylenoxid umgesetzten C₁₃-/C₁₅-Oxoalkohols zum entsprechenden Polyethersulfonat umgesetzt. Es resultierten 218 g Produkt, entsprechend 100 % Ausbeute, in Form einer pastösen Masse.

### Beispiel 3

Analog zu Beispiel 1 wurden 116 g (0,4 mol) eines mit 3 mol Ethylenoxid umgesetzten C₁₀-Oxoalkohols zum entsprechenden Polyethersulfonat umgesetzt. Es resultierten 196 g Produkt, entsprechend 100 % Ausbeute, in Form einer gelben Paste.

### Beispiel 4

Analog zu Beispiel 1 wurden 129 g (0,4 mol) eines mit 3 mol Ethylenoxid umgesetzten C₁₃-Oxoalkohols zum entsprechenden Polyethersulfonat umgesetzt. Es resultierten 208 g Produkt, entsprechend 100 % Ausbeute, in Form einer hellbraunen Paste.

### Beispiel 5

Analog zu Beispiel 1 wurden 164 g (0,4 mol) eines mit 5 mol Ethylenoxid umgesetzten C₁₃-Oxoalkohols zum entsprechenden Polyethersulfonat umgesetzt. Es resultierten 243 g Produkt, entsprechend 100 % Ausbeute, in Form einer braunen Paste.

### Anwendungstechnische Prüfungen

### A. Tensidgrunddaten

Als Anwendungseigenschaften der Vinylpolyetheralkohole I und der Polyethersulfonate III wurden die Oberflächenspannung, das Schaumvermögen und das Netzvermögen von wäßrigen Zubereitungen, die die Produkte aus den Beispielen 1 bis 5 enthielten, untersucht.

Die Oberflächenspannung wurde nach DIN 53 914 bestimmt. Dabei wird die Kraft in mN/m gemessen, welche notwendig ist, um einen horizontal aufgehängten Ring oder Bügel aus der Flüssigkeitsoberfläche herauszuziehen.

Das Schaumvermögen wurde nach DIN 53 902 durch Messung des Schaumvolumens in ml eine Minute nach Beendigung der Schaumerzeugung bestimmt.

Das Netzvermögen wurde nach DIN 53 901 durch Tauchen eines Baumwollgewebes in die zu untersuchende Tensidlösung ermittelt. Dabei wird die Zeit in sec gemessen, nach der das Gewebe seinen durch die eingeschlossene Luft verursachten Auftrieb verliert und zu sinken beginnt. Je kürzer die Zeitspanne, desto besser ist das Netzvermögen.

Tabelle 1 zeigt die Ergebnisse mit den Produkten der Beispiele 1 bis 5. Für die Bestimmung der Oberflächenspannung wurde jeweils eine wäßrige Lösung mit 0,1 g wasserfreier Wirksubstanz pro Liter verwendet, das Netzvermögen wurde in einer wäßrigen Lösung mit jeweils 1,0 g wasserfreier Wirksubstanz pro Liter ermittelt.

**Tabelle 1**

| Oberflächenspannung, Schaumvermögen und Netzvermögen von Vinylpolyetheralkoholen und Polyethersulfonaten | | | |
|---|---|---|---|
| Produkt gemäß | Oberflächenspannung bei 20°C (mN/m) | Schaumvermögen (ml) | Netzvermögen bei 25°C (sec) |
| Vinylpolyetheralkohol | | | |
| Beispiel 1 | 28,8 | 20 | 200 |

| Polyethersulfonat | | | |
|---|---|---|---|
| Beispiel 1 | 28,4 | 170 | 35 |
| Beispiel 2 | 33,2 | 120 | 125 |
| Beispiel 3 | 34,7 | 110 | 187 |
| Beispiel 4 | 28,8 | 200 | 21 |
| Beispiel 5 | 28,1 | 400 | 16 |

| zum Vergleich: | | | |
|---|---|---|---|
| Polyethersulfat* | 42,1 | >800 | 45 |

| | | | |
|---|---|---|---|
| * der Formel C₁₂H₂₅-/C₁₄H₂₉-O-(CH₂CH₂O)_{2,5}-SO₃)Na gemäß (6) | | | |

Aus Tabelle 1 ersieht man, daß die Vinylpolyetheralkohole und Polyethersulfonate Vorteile bei der Absenkung der Oberflächenspannung aufweisen und deutlich weniger schäumen, was für alle technischen Reinigungsprozesse mit hoher mechanischer Belastung vorteilhaft ist. Die Netzwerte dieser Produkte sind zum Teil besser, zum Teil schlechter als der Stand der Technik, je nach Ethylenoxid-Kettenlänge und Alkoholrest.

### B. Waschvermögen

Zur Bestimmung des Primärwaschvermögens (Schmutzentfernung) wurden die Polyethersulfonate in einer Testwaschmittelformulierung eingesetzt, mit der verschiedene Schmutzgewebe gewaschen wurden. Eine Erhöhung der Remissionswerte (Weißgrade) zeigt eine Verbesserung der Primärwaschwirkung an.

Die Waschversuche wurden im Launder-0-meter der Firma Atlas durchgeführt.

Folgende Waschbedingungen wurden gewählt:

| | |
|---|---|
| Anzahl der Wäschen: | jeweils drei |
| Temperatur: | 60°C und 30°C |
| Wasserhärte: | 16,8° dH ≙3 mmol/l (Ca:Mg = 4 : 1) |
| Waschdauer: | 30 min |
| Waschmittelkonz.: | 5 g/l |
| Flottenverhältnis: | 1 : 25 |
| Schmutzgewebe: | WFK 10 D (Testgewebe der Wäschereiforschung Krefeld, angeschmutzt mit einer Hautfett/Pigment-Mischung) |
| | EMPA 104 (Testgewebe der Eidgenössischen Materialprüfanstalt St. Gallen, angeschmutzt mit einer Mineralöl/Pigment-Mischung) |

Waschmittelformulierung:
30 % Polyethersulfonat gemäß den Beispielen 1 bis 5 als Tensid
15 % Kalikokosseife
1 % Polypropylenglykol (Molare Masse 600)
1 % Ethanol
Rest auf 100 % Wasser
Die Remissionswerte wurden durch photometrische Messung im Elrepho der Firma Zeiss an den Schmutzgeweben bestimmt.

Tabelle 2 zeigt die Ergebnisse der Versuche. Den Waschergebnissen ist zu entnehmen, daß die Polyethersulfonate gegenüber dem Stand der Technik teilweise deutliche Verbesserungen beim Primärwaschvermögen aufweisen.

**Tabelle 2**

| Waschvermögen von Polyethersulfonaten | | | | |
|---|---|---|---|---|
| Produkt gemäß | Primärwaschvermögen in % Remission | | | |
| | Gewebe WFK 10D | | Gewebe EMPA 104 | |
| | 60°C | 30°C | 60°C | 30°C |
| Beispiel 1 | 59.7 | 59.2 | 24.7 | 19.9 |
| Beispiel 2 | 64.7 | 59.2 | 23.7 | 19.2 |
| Beispiel 3 | 51.6 | 49.6 | 19.4 | 15.0 |
| Beispiel 4 | 63.7 | 58.8 | 22.2 | 18.5 |
| Beispiel 5 | 60.9 | 58,6 | 24.6 | 19.6 |

| zum Vergleich: | | | | |
|---|---|---|---|---|
| Polyethersulfat* | 57.4 | 55.2 | 22.1 | 17.7 |
| hydroxylgruppenfreies Polyethersulfonat** | 52.4 | 48.4 | 19.0 | 16.5 |
| Gewebe vor der Wäsche | 45.0 | 45.0 | 12.9 | 12.9 |

| | | | | |
|---|---|---|---|---|
| * der Formel C₁₂H₂₅-/C₁₄H₂₉-O-(CH₂CH₂O)_{2,5}-SO₃Na gemäß (6) | | | | |
| ** der Formel C₁₀H₂₁-O-(CH₂CH₂O)₃-CH₂CH₂SO₃Na gemäß (1) | | | | |

## Patentansprüche

1. Vinylpolyetheralkohole der allgemeinen Formel I 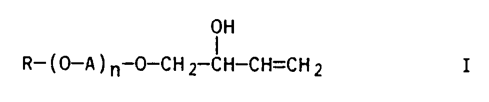 in der
R für C₁-C₂₅-Alkyl-, C₂-C₂₅-Alkenyl- oder Alkylarylgruppen mit insgesamt bis zu 20 C-Atomen mit Ausnahme der Bedeutung Vinyl steht,
A eine 1,2-Alkylengruppe mit 2 bis 4 C-Atomen bezeichnet und
n eine Zahl von 1 bis 20 bedeutet.

2. Vinylpolyetheralkohole I nach Anspruch 1, bei denen
R für C₈-C₂₀-Alkylgruppen steht,
A die 1,2-Ethylengruppe bezeichnet und
n eine Zahl von 2 bis 15 bedeutet.

3. Verfahren zur Herstellung von Vinylpolyetheralkoholen I gemäß Anspruch 1 oder 2, dadurch gekennzeichnet, daß man Polyetheralkohole der allgemeinen Formel II
R-(O-A)ₙ-OH II
mit Vinyloxiran in Gegenwart einer Base umsetzt.

4. Verfahren zur Herstellung von Polyethersulfonaten der allgemeinen Formel III 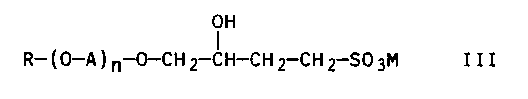 in der M für Wasserstoff, ein Alkalimetall oder Ammonium steht, dadurch gekennzeichnet, daß man Vinylpolyetheralkohole I gemäß Anspruch 1 oder 2 mit einem Alkalimetall- oder Ammoniumsulfit, -hydrogensulfit oder -disulfit oder Mischungen hiervon umsetzt.

5. Verfahren zur Herstellung von Polyethersulfonaten III nach Anspruch 4, dadurch gekennzeichnet, daß man Polyetheralkohole II mit Vinyloxiran in Gegenwart einer Base umsetzt und anschließend die entstandenen Vinylpolyetheralkohole I mit einem Alkalimetall- oder Ammoniumsulfit, -hydrogensulfit oder -disulfit oder Mischungen hiervon umsetzt.

6. Oberflächenaktive Zubereitungen, enthaltend 1 bis 50 Gew.-% eines Vinylpolyetheralkohols I gemäß Anspruch 1 oder 2 als oberflächenaktive Verbindung.

7. Verwendung von Vinylpolyetheralkoholen I gemäß Anspruch 1 oder 2 als oberflächenaktive Verbindungen.

## Claims

1. A vinyl polyether alcohol of the general formula I 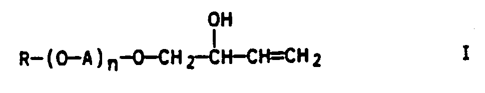 where
R is C₁-C₂₅-alkyl, C₂-C₂₅-alkenyl or alkylaryl having a total of up to 20 C atoms, excluding the meaning vinyl,
A is a 1,2-alkylene group having 2 to 4 C atoms and
n is a number from 1 to 20.

2. A vinyl polyether alcohol I as claimed in claim 1, where
R is C₈-C₂₀-alkyl,
A is the 1,2-ethylene group and
n is a number from 2 to 15.

3. A process for preparing vinyl polyether alcohols I as claimed in claim 1 or 2, which comprises reacting polyether alcohols of the general formula II
R-(O-A)ₙ-OH II
with vinyloxirane in the presence of a base.

4. A process for preparing polyether sulfonates of the general formula III 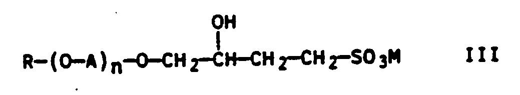 where M is hydrogen, an alkali metal or ammonium, which comprises reacting vinyl polyether alcohols I as claimed in claim 1 or 2 with an alkali metal or ammonium sulfite, hydrogen sulfite or disulfite or mixtures thereof.

5. A process for preparing polyether sulfonates III as claimed in claim 4, wherein polyether alcohols II are reacted with vinyloxirane in the presence of a base and the resulting vinyl polyether alcohols I are then reacted with an alkali metal or ammonium sulfite, hydrogen sulfite or disulfite or mixtures thereof.

6. A surface-active preparation, containing from 1 to 50 % by weight of a vinyl polyether alcohol I as claimed in claim 1 or 2 as surface-active compound.

7. The use of vinyl polyether alcohols I as claimed in claim 1 or 2 as surface-active compounds.

## Revendications

1. Polyétheralcools vinyliques de formule générale I 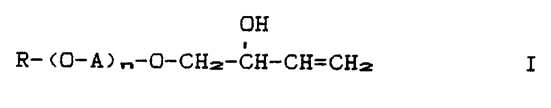 dans laquelle
R est mis pour un groupement alkyle en C₁-C₂₅, alcényle en C₂-C₂₅ ou alkylaryle renfermant au total jusqu'à 20 atomes de carbone, à l'exception de la signification d'un groupement vinyle,
A représente un groupement 1,2-alkylène à 2-4 atomes de carbone et
n est mis pour un nombre de 1 à 20.

2. Polyétheralcools vinyliques selon la revendication 1, dans lesquels
R est mis pour un groupement alkyle en C₈-C₂₀,
A représente le groupement 1,2-éthylène et
n est mis pour un nombre de 2 à 15.

3. Procédé de préparation de polyétheralcools vinyliques selon la revendication 1 ou 2, caractérisé en ce que l'on fait réagir des polyétheralcools de formule générale II
R-(O-A)ₙ-OH II
avec du vinyloxiranne en présence d'une base.

4. Procédé de préparation de polyéthersulfonates de formule générale III 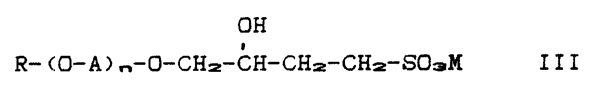 dans laquelle M est mis pour un atome d'hydrogène, de métal alcalin ou pour un groupement ammonium, caractérisé en ce que l'on fait réagir des polyétheralcools vinyliques I selon la revendication 1 ou 2 avec un sulfite, un hydrogénosulfite ou un disulfite de métal alcalin ou d'ammonium ou avec des mélanges de ceux-ci.

5. Procédé de préparation de polyéthersulfonates III selon la revendication 4, caractérisé en ce que l'on fait réagir des polyétheralcools II avec du vinyloxiranne en présence d'une base, puis on fait réagir les polyétheralcools vinyliques I formés avec un sulfite, un hydrogénosulfite ou un disulfite de métal alcalin ou d'ammonium ou avec des mélanges de ceux-ci.

6. Préparations tensio-actives, contenant 1 à 50% en poids d'un polyétheralcool vinylique I selon la revendication 1 ou 2 en tant que composé tensio-actif.

7. Utilisation de polyétheralcools vinyliques I selon la revendication 1 ou 2 comme composés tensio-actifs.
